# EUROPEAN PATENT SPECIFICATION

(11) **EP 3 011 016 B1**
(45) Date of publication and mention of the grant of the patent: **07.08.2019**
(21) Application number: 14814177.3
(22) Date of filing: 18.06.2014
(51) Int. Cl.: C12N 5/095, C12N 5/09, G01N 33/50

(54) **METHOD OF CULTURING CANCER STEM CELLS**
VERFAHREN ZUR KULTIVIERUNG VON KREBSSTAMMZELLEN
PROCÉDÉ DE CULTURE DE CELLULES SOUCHES CANCÉREUSES

(30) Priority: 18.06.2013 SG 201304704
(43) Date of publication of application: 27.04.2016
(73) Proprietor: Agency for Science, Technology and Research, Singapore 138632 (SG)
(72) Inventor: KURISAWA, Motoichi, Singapore 138669 (SG); YAMASHITA, Atsushi, Singapore 138669 (SG)
(74) Representative: Germinario, Claudio
(86) International application number: PCT/SG2014/000290
(87) International publication number: WO 2014/204406

(56) References cited:
- WO-A1-2010/126452
- WO-A1-2010/138082
- WO-A2-2006/010066
- WO-A2-2009/116951
- WO-A2-2011/059325
- WO-A2-2011/059326
- WO-A2-2011/059326
- JP-A- 2007 297 360
- US-A1- 2012 177 604
- US-A1- 2012 288 564
- XU KEMING ET AL: "Injectable hyaluronic acid-tyramine hydrogels incorporating interferon-[alpha]2a for liver cancer the", JOURNAL OF CONTROLLED RELEASE, ELSEVIER, AMSTERDAM, NL, vol. 166, no. 3, 14 January 2013 (2013-01-14), pages 203-210, XP028994979, ISSN: 0168-3659, DOI: 10.1016/J.JCONREL.2013.01.008
- TOH, W.S. ET AL.: 'Modulation of mesenchymal stem cell chondrogenesis in a tunable hyaluronic acid hydrogel microenvironment' BIOMATERIALS vol. 33, 2012, pages 3835 - 3845, XP028468794
- DESAI, N. ET AL.: 'Three dimensional culture of fresh and vitrified mouse pre-antral follicles in a hyaluronan-based hydrogel: a preliminary investigation of a novel biomaterial for in vitro follicle maturation' REPRODUCTIVE BIOLOGY AND ENDOCRINOLOGY vol. 10, 2012, pages 1 - 12, XP021095725
- JIN, R. ET AL.: 'Tyrosinase-mediated in situ forming hydrogels from biodegradable chondroitin sulfate-tyramine conjugates' POLYMER INTERNATIONAL vol. 62, March 2013, pages 353 - 361, XP055300486

## Description

### Technical Field

The present invention generally relates to a method for culturing a population of cancer stem cells. The present invention also relates to use of a gel as a cell culture substrate.

### Background

Cancer stem cells (CSCs) are a subpopulation of cancer cells (found within tumors or hematological cancers) that are similar to normal stem cells in the sense that they are able to give rise to all cell types found in a particular cancer. This includes characteristics such as self-renewal, propagation of cancer or differentiation into the various types of cancer cells. Such cells are believed to be able to persist in tumors as a distinct population, causing cancer relapse and metastasis due to the formation of new tumors.

One of the key challenges in cancer therapy would be the eradication of CSCs. Poor prognosis and high mortality of cancer patients are believed to be caused by properties of CSCs including self-renewal, metastasis, chemo- and radioresistance. Conventional anti-cancer drugs can kill the bulk of tumor cells but ultimately fail to eradicate CSCs. The surviving CSCs propagate and regenerate new tumor cells, which are closely linked to the patient's poor prognosis and relapse. To develop effective methods to eradicate CSCs and screen new anti-cancer drugs, much efforts have been spent on identifying and characterizing the CSCs by fluorescence-activated cell sorting (FACS) of cancer cells labeled with different monoclonal antibodies. CD44 is one of the most commonly studied CSC markers. The subpopulation of cancer cells with CD44^{high}/CD24^{low}, CD44^{high}/CD133^{high}, CD44^{high}/aldehyde dehydrogenase 1 family, member A1 (ALDH1A1)^{high} and CD44^{high}/epithelial cell adhesion molecule (EpCAM)^{high} showed chemoresistance and tumorigenesis. However, such a population is generally small and unstable in culture, making it difficult to perform standard high through-put cell viability assays. In fact, the CSC enriched population, which was prepared by FACS sorting, rapidly decreased during *in vitro* culture.

There is a need to provide a method of culturing cancer stem cells that overcomes, or at least ameliorates, one or more of the disadvantages described above.

There is a need to provide a cell culture substrate that is able to support the selection, maintenance and propagation of cancer stem cells.

### Summary

According to a first aspect, there is provided a method for culturing a population of cancer stem cells comprising introducing the cancer stem cells on or in a cell culture substrate, wherein the cell culture substrate is in the form of a gel comprising a conjugate of a glycosaminoglycan and a substituted phenalkylamine.

Advantageously, cancer stem cells that contain a marker that interacts with the glycosaminoglycan may be selectively cultured on the gel. The expression level of the marker may control the propagation of the population of cancer cells on the gel.

The cancer stem cells may be present in a cancer cell line and hence the method for culturing cancer stem cells can also be used to culture a cancer cell line enriched in cancer stem cells that have a high expression of the marker. Hence, in one embodiment, the method for culturing a population of cancer stem cells may include a method for culturing a cancer cell line comprising introducing the cancer cell line on or in a cell culture substrate, wherein the cell culture substrate is in the form of a gel comprising a conjugate of a glycosaminoglycan and a substituted phenalkylamine.

The stiffness or crosslink density of the gel may be altered in order to promote the growth and maintenance of the cancer stem cells or cancer cell line. The stiffness or crosslink density of the gel may also affect the chemoresistance of the cancer stem cells or cancer cell line to a selected chemotherapeutic drug.

According to a second aspect, there is provided a method for selectively separating a population of cancer stem cells from a plurality of cancer cell lines, comprising the steps of: (a) subjecting the plurality of cancer cell lines to a cell culture substrate, wherein the cell culture substrate is in the form of a gel comprising a conjugate of a glycosaminoglycan and a substituted phenalkylamine; and (b) allowing the cancer stem cells to interact with the cell culture substrate to thereby separate the cancer stem cells from the plurality of cancer cell lines.

According to a third aspect, there is provided a method of screening drugs for a population of cancer stem cells comprising the step of culturing the cancer stem cells on or in a cell culture substrate, wherein the cell culture substrate is in the form of a gel comprising a conjugate of a glycosaminoglycan and a substituted phenalkylamine, and wherein the gel has a stiffness that is equal to or less than 100 kPa.

According to a fourth aspect, there is provided use of a gel comprising a conjugate of a glycosaminoglycan and a substituted phenalkylamine as a cell culture substrate.

According to a fifth aspect, there is provided use of a cell culture substrate in the form of a gel comprising a conjugate of a glycosaminoglycan and a substituted phenalkylamine having cancer stem cells cultured on or in the cell culture substrate for screening anti-cancer drugs.

### Definitions

The following words and terms used herein shall have the meaning indicated:
The terms "cancer stem cell marker" or "marker" are to be interpreted broadly to refer to genes and their protein products that can be used to isolate and identify the cancer stem cells.

The terms "selection" or "selectively" as well as grammatical variants thereof are to be interpreted broadly to refer to the isolation of a desired or target type of cancer cells or cancer stem cells from a plurality or mixture of various types of cancer cells, cancer stem cells or cancer cell lines.

The word "substantially" does not exclude "completely" e.g. a composition which is "substantially free" from Y may be completely free from Y. Where necessary, the word "substantially" may be omitted from the definition of the invention.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and subgeneric groupings falling within the generic disclosure also form part of the disclosure. This includes the generic description of the embodiments with a proviso or negative limitation removing any subject matter from the genus, regardless of whether or not the excised material is specifically recited herein.

### Detailed Disclosure of Embodiments

Exemplary, non-limiting embodiments of a method for culturing a population of cancer stem cells will now be disclosed.

The method comprises introducing the cancer stem cells on or in a cell culture substrate, wherein the cell culture substrate is in the form of a gel comprising a conjugate of a glycosaminoglycan and a substituted phenalkylamine.

The cancer stem cells may contain a marker (also known as a cancer stem cell marker).

The cancer stem cells may be present in a cancer cell line and hence the method for culturing cancer stem cells can also be used to culture a cancer cell line enriched in cancer stem cells that have a high expression of a cancer stem cell marker. Hence, in one embodiment, the method for culturing a population of cancer stem cells may include a method for culturing a cancer cell line comprising introducing the cancer cell line on or in a cell culture substrate, wherein the cell culture substrate is in the form of a gel comprising a conjugate of a glycosaminoglycan and a substituted phenalkylamine.

The expression level of the cancer stem cell marker may control the propagation of the population of cancer cells on the gel. The expression level of the cancer stem cell marker is defined as the number of cancer stem cell marker molecules in a single cell. The expression level may be expressed as an average value of relative expression level of the cancer stem cell marker, when compared to a comparative cell. The average relative expression level may be measured by flow cytometric analysis (FACS). When determining whether a cell (or cell line) has a "high" expression level of the cancer stem cell marker, the average value of the relative expression level of the cancer stem cell maker may be a value that is above 20, when compared to the comparative cell. The average value of the relative expression level of the cancer stem cell maker may be above 30, above 40, above 50, above 60, above 70, above 80, above 90, above 100, above 110 or above 150, when compared to a comparative cell. The expression level may also be obtained by determining the mRNA expression level of the cancer stem cell marker. As an example, the comparative cell may be BT-474 (ATCC® HTB-20™, obtained from ATCC of Manassas, Virginia of the United States of America).

The cancer stem cells may be incubated on the cell culture substrate. The incubation conditions may be at a temperature of about 36°C to about 37°C, at a duration of about 5 minutes to about 24 hours, and in a humidified atmosphere. As an example, the incubation conditions may be at 1 hour at 37°C in a humidified atmosphere of 5% carbon dioxide.

After incubation, any cancer cells that are not attached to the cell culture substrate may be removed. The unattached cells may be removed by washing the cells with a suitable buffer for a number of times. The cells may be washed with an exemplary buffer such as phosphate buffer saline (PBS) for 1 to 5 times, or 3 times. By washing the cells, cells that highly express markers such as CD44, EpCAM and other markers may be obtained.

The cancer stem cell marker may interact with the glycosaminoglycan present in the gel. The cancer stem cell marker may be a receptor for the glycosaminoglycan. The marker may be selected from the group consisting of CD44, Receptor for HA-mediated motility (RHAMM) and intracellular adhesion molecule-1 (ICAM-1).

When cultured on the gel, the growth and maintenance of the cancer stem cells may be determined by the mRNA expressions of Nanog, Sox-2 or EpCAM. For Nanog, the cancer stem cells may have a mRNA expression level that is at least 2 folds that of the same cells but grown on a polystyrene cell culture plate control. For Sox-2, the cancer stem cells may have a mRNA expression level that is at least 1.25 folds that of the same cells but a polystyrene cell culture plate control. For EpCAM, the cancer stem cells may have a mRNA expression level that is at least 2.25 folds that of the same cells but grown on a polystyrene cell culture plate control.

The stiffness or crosslink density of the gel may act as an alternative or additional control to the selection of cancer stem cells (or cancer cells) that can be cultured on the gel. The stiffness or crosslink density of the gel may also control the growth and/or maintenance of the cancer stem cells (or cancer cells) that do selectively grow on the gel. The stiffness or crosslink density of the gel may also affect the chemoresistance of the cancer stem cells (or cancer cells) to a selected chemotherapeutic drug.

The stiffness of the gel may be a value selected from a range of about 0.1 kPa to about 100 kPa, about 0.1 kPa to about 1 kPa, about 0.1 kPa to about 2 kPa, about 0.1 kPa to about 3 kPa, about 0.1 kPa to about 4 kPa, about 0.1 kPa to about 5 kPa, about 0.1 kPa to about 6 kPa, about 0.1 kPa to about 7 kPa, about 0.1 kPa to about 8 kPa, about 0.1 kPa to about 9 kPa, about 0.1 kPa to about 20 kPa, about 0.1 kPa to about 30 kPa, about 0.1 kPa to about 40 kPa, about 0.1 kPa to about 50 kPa, about 0.1 kPa to about 60 kPa, about 0.1 kPa to about 70 kPa, about 0.1 kPa to about 80 kPa, about 0.1 kPa to about 90 kPa, about 1 kPa to about 10 kPa, about 2 kPa to about 10 kPa, about 3 kPa to about 10 kPa, about 4 kPa to about 10 kPa, about 5 kPa to about 10 kPa, about 6 kPa to about 10 kPa, about 7 kPa to about 10 kPa, about 8 kPa to about 10 kPa, about 9 kPa to about 10 kPa, about 10 kPa to about 100 kPa, about 20 kPa to about 100 kPa, about 30 kPa to about 100 kPa, about 40 kPa to about 100 kPa, about 50 kPa to about 100 kPa, about 60 kPa to about 100 kPa, about 70 kPa to about 100 kPa, about 80 kPa to about 100 kPa, about 90 kPa to about 100 kPa, or about 5k Pa to 10k Pa. The stiffness of the gel may be about 0.1 kPa, 0.2 kPa, 0.4 kPa, 0.5 kPa, 1.0 kPa, 2.5 kPa or 4.0 kPa.

The crosslink density of the gel may be a value selected from the range of about 1x10⁻⁶ to about 1x10⁻³ mol/cm³, 1x10⁻⁵ to about 1x10⁻³ mol/cm³, 1x10⁻⁴ to about 1x10⁻³ mol/cm³, 1x10⁻⁶ to about 1x10⁻⁵ mol/cm³, or 1x10⁻⁶ to about 1x10⁻⁴ mol/cm³.

The storage modulus of the gel may be a value selected from a range of about 30 to about 100,000 Pa, about 30 to about 1,000 Pa, about 30 to about 10,000 Pa, about 30 to about 50,000 Pa, about 50,000 to about 100,000 Pa, about 1,000 to about 10,000 Pa, or about 10,000 to about 100,000 Pa.

The gel may be a hydrogel. The gel or hydrogel may be a conjugate of a glycosaminoglycan and a substituted phenalkylamine. The glycosaminoglycan may be a non-sulfated glycosaminoglycan such as hyaluronic acid (HA). The substituted phenalkylamine may be a substituted phenmethylamine, phenethylamine, phenpropylamine, phenbutylamine or phenpentylamine.

Where the phenalkylamine is phenethylamine, the phenethylamine may be tyramine such as a meta-tyramine or a para-tyramine. The gel may be an enzymatically cross-linked gel. The gel may be composed of a HA-tyramine conjugate, which may be formed using oxidative coupling of tyramine moieties catalyzed by catalysts such as hydrogen peroxide and horseradish peroxidase.

In the gel, the degree of substitution (defined as the number of substituted phenalkylamine molecules per 100 repeating units of glycosaminoglycan) may be a value selected from a range of about 1 to about 20, about 1 to about 5, about 1 to about 10, about 1 to about 15, about 5 to about 20, about 10 to about 20 or about 15 to about 20. The degree of substitution may be about 6.

When the stiffness of the cell culture substrate or gel is less than or equal to 100kPa, or 1.0 kPa, the cancer stem cells may become resistant to an anti-cancer drug. The cells may have at least 70% viability when in the presence of the anti-cancer drug. The anti-cancer drug may be cisplatin or doxorubicin.

There is also provided a method for selectively separating a population of cancer stem cells from a plurality of cancer cells, comprising the steps of:
a. subjecting the plurality of cancer cells to a cell culture substrate, wherein the cell culture substrate is in the form of a gel comprising a conjugate of a glycosaminoglycan and a substituted phenalkylamine; and
b. allowing the cancer stem cells to interact with the cell culture substrate to thereby separate the cancer stem cells from the plurality of cancer cells.

In order to allow the interaction between the cancer stem cells and the cell culture substrate, step (b) may comprise the step of binding the cancer stem cells to the glycosaminoglycan of the cell culture substrate via receptor-ligand binding. For receptor-ligand binding to occur, the cancer stem cells contain a marker that is a receptor for the glycosaminoglycan.

The expression level of the cancer stem cell marker may also affect the ability of the cancer stem cells (or cancer cell line containing such cancer stem cells) to bind with the glycosaminoglycan.

There is also provided a method of screening drugs for a population of cancer stem cells comprising the step of culturing said cancer stem cells on or in a cell culture substrate, wherein said cell culture substrate is in the form of a gel comprising a conjugate of a glycosaminoglycan and a substituted phenalkylamine, and wherein said gel has a stiffness that is equal to or less than 100 kPa. The stiffness may be equal to or less than 1.0 kPa, 0.5 kPa, 0.4 kPa, 0.2 kPa or 0.1 kPa.

There is also provided use of a gel comprising a conjugate of a glycosaminoglycan and a substituted phenalkylamine as a cell culture substrate.

The cell culture substrate may be selective for a population of cancer stem cells. The cancer stem cells may express a marker that may interact with the glycosaminoglycan via receptor-ligand binding.

There is also provided use of a cell culture substrate in the form of a gel comprising a conjugate of a glycosaminoglycan and a substituted phenalkylamine having cancer stem cells cultured on or in the cell culture substrate for screening anti-cancer drugs.

The anti-cancer drug to be screened may not be particularly limited and may include cisplatin or doxorubicin as well as any other anti-cancer drugs.

### Brief Description Of Drawings

The accompanying drawings illustrate a disclosed embodiment and serves to explain the principles of the disclosed embodiment. It is to be understood, however, that the drawings are designed for purposes of illustration only, and not as a definition of the limits of the invention.
Fig. 1 is a schematic diagram showing a gel for supporting the selection and culture of a population of cancer cells that contain a cancer stem cell marker.
Fig. 2(a) is a graph showing the storage modulus of a gel at various concentrations of hydrogen peroxide. The results are shown as average values ± standard deviation (*n* = 3). Fig. 2(b) is a graph showing the cross link density and molecular weight between cross-links of HA-Tyr hydrogel (1.75 % (w/v)) prepared with varying concentrations of hydrogen peroxide. The results are shown as average values ± standard deviation (*n* = 3 - 5).
Fig. 3 is a number of graphs showing the flow cytometric analysis (FACS) of the amount of cancer cells that contain the cancer stem cell marker in (a) MDA-MB-231; (b) MCF-7; and (c) BT-474 cancer cell lines. Isotype controls were conducted for all cell lines.
Fig. 4(a) is a graph showing the FACS of MCF-7, HCC1937 and MDA-MB-231 cells. Fig. 4(b) is a bar graph showing the relative mRNA expression levels of CD44 in MCF-7, HCC1937 and MDA-MB-231 cells as evaluated by real time RT-PCR. Results are shown as average values + standard deviation (n = 3) (*P ≤ 0.005).
Fig. 5 is a number of graphs showing the adhesion of (a) MCF-7; (b) HCC1937; (c) MDA-MB-231; and (d) BT-474 cancer cells on the gel as a function of incubation time. The results are shown as average values ± standard deviation (*n* = 4).
Fig. 6(a) shows a number of phase contrast microscopic images of MDA-MB-231 cells adhered to polystyrene control and gels of varying stiffness at 24 hours after cell seeding.
Fig. 6(b) is a graph of a quantitative analysis of the cell spreading area on a polystyrene control and gels of varying stiffness. The results are shown as average values ± standard deviation (*n* = 25).
Fig. 7 is a graph showing the cell proliferation of MDA-MB-231 on gels of varying stiffness as a function of incubation time. The results are shown as average values ± standard deviation (*n* = 4). **P* ≤ 0.01.
Fig. 8(a) are a series of phase contrast microscopic images of MDA-MB-231 cells and their colonies on HA-Tyr hydrogels (I-V, VII) and polystyrene (VI) at 14 days after cell seeding. The stiffness of the various hydrogels are 0.1 kPa (I) and (VII), 0.2 kPa (II), 0.5 kPa (III), 1 kPa (IV), 4 kPa (V). The scale used in (I) is 300 *µ*m while that in (VII) is 100 *µ*m. Fig. 8(b) shows the size of the colonies as measured from phase contrast microscopic images using Image-Pro plus image software analysis software. Results are shown as the average values + standard deviation (n = 4)(*P ≤ 0.001).
Fig. 9 is a graph showing the relative mRNA expression levels of Nanog, Sox-2 and EpCAM in MDA-MB-231 cells on a polystyrene control and gels of varying stiffness, whereby the mRNA expression levels were evaluated by real time RT-PCR. The results are shown as average values ± standard deviation (*n* = 3).
Fig. 10(a) is a graph showing the relative mRNA expression levels of CD44 (CD44s, CD44v3-10 and CD44v8-10). Fig. 10(b) is a graph showing the relative mRNA expression levels of CSC markers such as Sox-2, EpCAM and ALDH1A1 in MDA-MB-231 cells on HA-Tyr hydrogels evaluated by real time RT-PCR. The results are shown as average values ± standard deviation (*n* = 3) (*P ≤ 0.05, **P ≤ 0.01, ***P ≤ 0.001).
Fig. 11 is a graph showing the prevention of MDA-MB-231 cell adhesion on HA-Tyr hydrogel due to the presence of blocking antibodies against CD44.
Fig. 12(a) is a graph showing the relative mRNA expression levels of CD44 (CD44s, CD44v3-10, CD44v8-10), EpCAM and ALDH1A1 in MDA-MB-231 cells, which were selected after 1 hour cell seeding by 3 times wash with PBS. Fig. 12(b) is a graph showing the relative mRNA expression levels of CD44v8-10 in MDA-MB-231 cells, which were selected after 1 hour cell seeding by 3 times wash with PBS, on HA-Tyr hydrogels after 14 day. mRNA expression levels were evaluated by real time RT-PCR. The results are shown as average values ± standard deviation (*n* = 3) (*P ≤ 0.05, **P ≤ 0.01).
Fig. 13(a) is a graph showing the viability of the MDA-MB-231 cells in the presence of cisplatin. The results are shown as average values ± standard deviation (*n* = 4).
Fig. 13(b) is a graph showing the viability of the MDA-MB-231 cells in the presence of doxorubicin. The results are shown as average values ± standard deviation (*n* = 4) .

### Detailed Description of Drawings

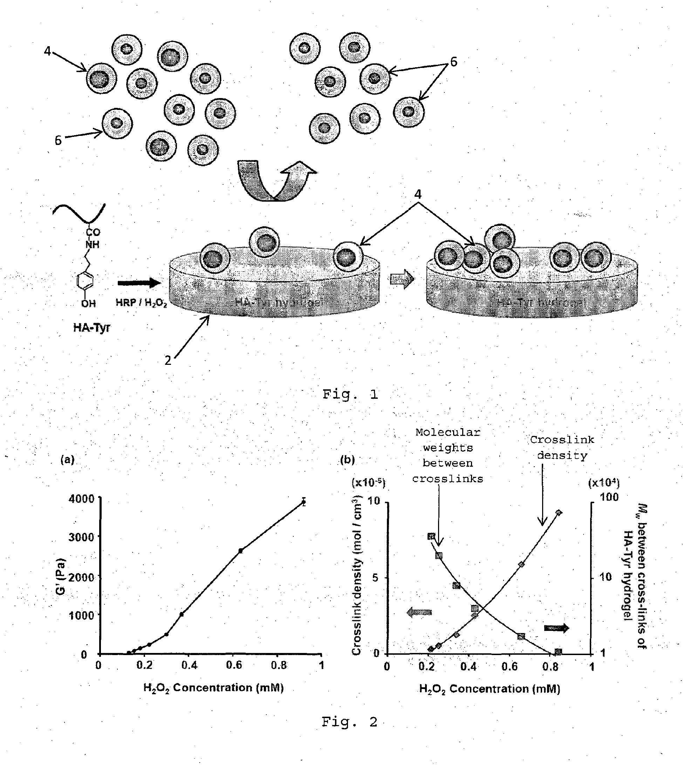

Referring to Fig. 1, there is provided a schematic diagram showing a gel 2 for supporting the selection and culture of a population of cancer cells that contain a cancer stem cell marker 4.

In Fig. 1, the gel 2, composed of a HA-tyramine conjugate, was formed using oxidative coupling of tyramine moieties catalyzed by hydrogen peroxide (H₂O₂) and horseradish peroxidase (HRP). When a plurality of cancer cells (4, 6) are exposed to the gel 2, only those cells that have a high expression level of the cancer stem cell marker 4 will adhere to the gel 2. Cells that do not have a high expression level of the cancer stem cell marker 6 will not adhere to the gel 2. Hence, by culturing the plurality of cancer cells in the presence of the gel 2, this enables the selection and separation of the highly expressing cancer cells 4 from the low expressing cancer cells 6.

### Examples

Non-limiting examples of the invention will be further described in greater detail by reference to specific Examples, which should not be construed as in any way limiting the scope of the invention.

Sodium hyaluronate (HA) (Mw=90 kDa, Density=1.05 g/cm³) was kindly donated by JNC Corporation (Tokyo, Japan). Tyramine hydrochloride (Tyr·HCl), N-hydroxysuccinimide (NHS), 1-ethyl-3-(3-dimethylaminopropyl)-carbodiimide hydrochloride (EDC·HCl), hyaluronidase from bovine testes (400-1,000 units/mg) and cisplatin were all purchased from Sigma-Aldrich (Minnesota of the United States of America). Horseradish peroxidase (HRP, 100 units/mg) was purchased from Wako Pure Chemical Industries (Osaka, Japan). Hydrogen peroxide (H₂O₂) was obtained from Lancaster. Doxorubicin was obtained from Boryung pharmaceutical (Seoul, South Korea). AlamarBlue, CyQUANT® cell proliferation assay kit, TRIzol® and Taqman® gene expression master mix were provided by Life Technologies (Singapore), respectively. Mouse monoclonal antibody to human CD44 and FITC-conjugated rat antimouse IgG2a secondary antibody were obtained from GeneTex (Hsinchu, Taiwan). Rat monoclonal anti-CD44 antibody (Hermes-1) were purchased from Abcam (Cambridge, United Kingdom). Trypsin-EDTA (0.025%) and penicillin/streptomycin were purchased from PAN Biotech GmbH (Aidenbach, Germany). Heat-inactivated fetal bovine serum (FBS) was purchased from GE Healthcare (Buckinghamshire, United Kingdom). Phosphate buffered saline (PBS, 150 mM, pH 7.3), Dulbecco's modified eagle medium (DMEM) and RPMI-1640 medium were all supplied by the media preparation facility in Biopolis (Singapore).

MDA-MB-231, MCF-7, HCC1937 and BT-474 breast cancer cell lines were purchased from the American Type Culture Collection (Manassas, Virginia of the United States of America). MDA-MB-231, HCC1937 and BT-474 cells were grown in RPMI-1640 medium containing 10% FBS and 1% penicillin/streptomycin. MCF-7 cells were grown in DMEM containing 10% FBS and 1% penicillin/streptomycin. All cell lines were cultured on polystyrene tissue culture flask, and were passaged when reach to 80% confluent.

Statistical analysis: All data were expressed as mean + standard deviation (SD). Unless otherwise stated, differences between the values were assessed using Student's *t* test using SigmaStat software (Systat Software, Inc.); *p* < 0.05 was considered statistically significant. All of the experiments were performed in triplicate.

### Example 1

### Synthesis of HA-tyramine Gel

An enzymatically cross-linked HA gel, composed of HA-tyramine conjugate, was formed using oxidative coupling of tyramine moieties catalyzed by H₂O₂ and HRP. Here, solutions of HRP and H₂O₂ with different concentrations (ranging from 0.22 mM to 1.15 mM of H₂O₂) were added to HA-Tyr conjugate (3% w/v, 175 µl in PBS). An appropriate amount of PBS was added to the mixture so that the total volume of the mixture was 300 µl. The final concentration of HA-Tyr conjugates and HRP were 1.75% (w/v) and 0.125 units/ml, respectively. The mixture was vortex immediately.

The degree of substitution (the number of tyramine molecules per 100 repeating units of HA) was 6 as determined by ¹H NMR.

The stiffness and gelation rate of the gel could be independently tuned by H₂O₂ and HRP concentrations, respectively.

Rheological measurements of the gels were performed with a HAAKE Rheoscope 1 rheometer (Karlsruhe, Germany) using a cone and plate geometry of 3.5 cm diameter and 0.949° cone angle. The measurements were taken at 37°C in the dynamic oscillatory mode with a constant deformation of 1% and a frequency of 1 Hz. To avoid slippage of samples during the measurement, a roughened glass bottom plate was used. 250 µl of the gel mixture prepared as above was applied to the bottom plate of the rheometer. The upper cone was then lowered to a measurement gap of 0.025 mm and a layer of silicon oil was carefully applied around the cone to prevent solvent evaporation during the experiment. Rheological measurement was allowed to proceed until the storage modulus (G') reached a plateau.

As seen in Fig. 2(a), G' of the HA-Tyr gel was well controlled by H₂O₂ concentration, and ranged from 70 to 4,000 Pa when an aqueous solution of HA-Tyr conjugate (1.75 % (w/v)) was utilized. G' increased with increasing H₂O₂ concentration from 0.22 mM to 1.15 mM, suggesting that a higher crosslinking density was achieved when the H₂O₂ concentration increased.

The average molecular weight between crosslinks (*M_{c}*) and crosslinking density (*vₑ*) of the HA-Tyr hydrogels were determined. From the results of the storage modulus (*G'*) measurement, the average molecular weight between crosslinks (*M_{c}*) and crosslinking density (*vₑ*) were calculated by rubber-elasticity theory. As shown in Fig. 2(b), the *M_{c}* of the HA-Tyr hydrogels decreased with increasing H₂O₂ concentration, while the *vₑ* of the HA-Tyr hydrogels increased with increasing H₂O₂ concentration.

To evaluate the cancer cell adhesion (Example 2), proliferation (Example 3), maintenance (Example 4), inhibition of HA-CD44 interaction (Example 5) and chemoresistance (Example 6), a number of gels of varying stiffness (0.1, 0.2, 0.5, 1.0, 2.5 and 4.0 kPa, where appropriate) were used. As will be seen from the Examples below, breast cancer cell adhesion on HA-Tyr hydrogel was strongly modulated by the stiffness of the hydrogels, and was dependent on CD44-HA interaction. HA-Tyr hydrogels, as compared to polystyrene, provided different culture environments in maintaining the round shape cell morphology, low cell proliferation and colony formation. The HA-Tyr hydrogels outperformed the polystyrene with better enhancement of CD44 variant isoforms, Sox-2 and ALDH1A1 mRNA expression levels. The mechanical properties of the hydrogel (such as the components and stiffness of the hydrogel) as well as expression levels of CD44 variant isoforms are factors that affect the cell adhesion, proliferation and malignancy of the cancer cells. Controlling the stiffness of the HA-Tyr -hydrogel is a simple and effective means to change the malignancy of breast cancer cells.

### Example 2

### Cancer Cell Adhesion

Four types of breast cancer cell lines such as MDA-MB-231, MCF-7, HCC1937 and BT-474 were investigated. In order to examine the population of CD44 positive cells in these cancer cell lines, flow cytometric analysis (FACS) of CD44 surface expression were carried out.

In FACS, cells were washed with PBS and then harvested by using trypsin-EDTA. The detached cells were then washed with PBS containing 2% FBS, and were incubated with anti-CD44 antibody on ice for 30 minutes. After washing with PBS containing 2% FBS, secondary antibody, which was conjugated with fluorescein isothiocyanate (FITC), were added to the cell suspensions at the concentrations recommended by the manufacture and incubated on ice in the dark for 30 minutes. The cells were then washed twice and resuspended in 0.5 ml of PBS containing 2% FBS. The cells were then analyzed and sorted using a BD LSRII flow cytometry analyser (BD Biosciences, of New Jersey of the United States of America).

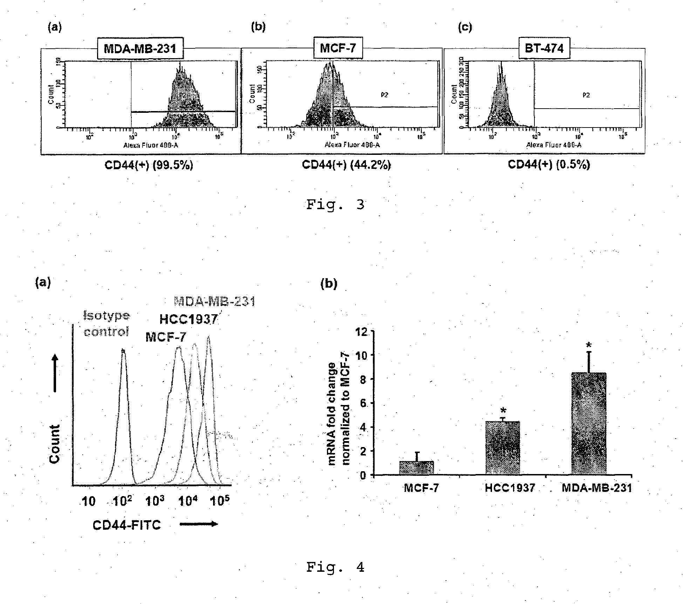

As shown in Fig. 3, it can be seen that 99.5%, 44.2% and 0.5% of CD44 positive cells were detected in MDA-MB-231 (Fig. 3(a)), MCF-7 (Fig. 3(b)) and BT-474 (Fig. 3(c)), respectively, using FACS. The CD44 protein expression levels (average) of MDA-MB-231, HCT116 and MCF-7 cells were 103, 12 and 5 times higher than that on BT-474 cells.

In addition, when comparing between MDA-MB-231, MCF-7, HCC1937, it can be seen that there is a high expression level of CD44 protein on MDA-MB-231 cells, middle expression level on HCC1937 cells and lower expression levels on MCF7 cells detected using FACS (Fig. 4(a)). The CD44 expression level is defined as the number of CD44 molecules in a single cell. Different expression levels of CD44 among these breast cancer cell lines were evaluated based on protein expression level on cell surfaces (Fig. 4(a)) as well as mRNA expression level (Fig. 4(b)). The mRNA expression levels of CD44 in MDA-MB-231 and HCC1937 cells were 8.5 and 4.5 times higher than that of MCF-7 cells. These results were closely related to the results of the CD44 protein expression levels as shown in Fig. 4(a).

The cancer cell adhesion of these cell lines to the surface of the HA-Tyr gels were then investigated. HA-Tyr gels (250 *µ*l) with different stiffness (0.1, 0.2, 0.5, 1.0, 2.5 and 4.0 kPa, where appropriate) were prepared in a 24-well plate. The gels were allowed to settle overnight. Gels were then washed three times with PBS and once with culture medium. 250 *µ*l of breast cancer cells in culture medium at cell density of 1.0 × 10⁵ cells/ml was seeded onto the gels.

The plates were returned to an incubator (at 37°C in a humidified atmosphere of 5% CO₂) for an appropriate period of time ranging from 1 to 9 hours. At selected time intervals, the media with unattached cells were aspirated and the wells were washed three times with PBS. A cell culture plate without the gel served as a comparison.

For MDA-MB-231, MCF-7 and HCC1937, quantification of DNA was performed using CyQUANT® cell proliferation assay kit to evaluate the number of attached cells. The recommended manufacturer's protocol for assay was followed. The cells on the HA-Tyr hydrogels and polystyrene were harvested by incubating with hyaluronidase (1,000 units/ml) and trypsin-EDTA, respectively. The cell pellets were wahed with PBS twice and lysed by a freeze-thaw cycle. Then, samples were dissolved in 200 µl of CyQUANT working solution. The number of cells was then determined by fluorescence measurement of the sample solution along with the known density of cell suspension based on a standard curve. The fluorescence measurement was performed using a microplate reader with excitation and emission at 480 and 520 nm, respectively.

For BT-474, the cells attached to the gels were incubated in culture medium containing 10% Alamar Blue dye at 37°C for 4 hours. The fluorescence measurement of the Alamar Blue dye was performed using a microplate reader with excitation and emission at 545 and 590 nm, respectively. Then, 100 *µ*l of AlamarBlue solution was transferred to 96 well plate to measure the fluorescence intensity. The fluorescence intensity of the Alamar blue dye was regarded as the number of attached cells in this study.

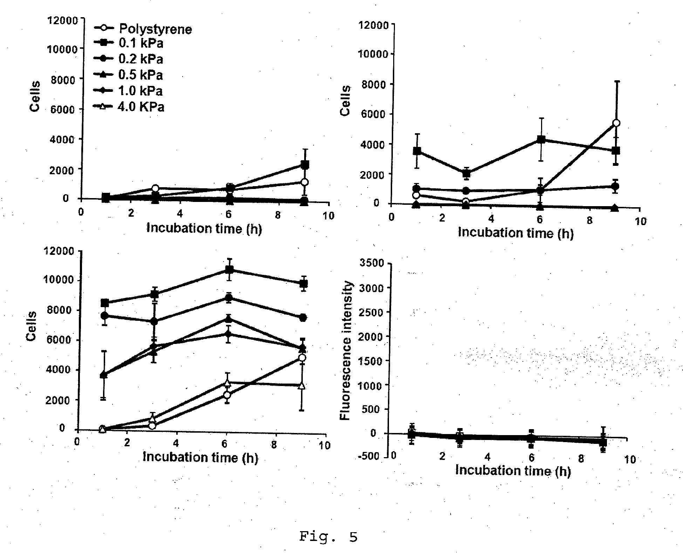

Fig. 5 shows the cell adhesion on the surface of HA-Tyr gels. A significant number of MDA-MB-231 cells were attached to the surfaces of HA-Tyr gels (Fig. 5(c)). The attached MDA-MB-231 cell number was dependent on HA-Tyr hydrogel stiffness, and the attached cell number increased with decreasing hydrogel stiffness. Fig. 5(a) and Fig. 5(b) also show that the cell adhesion of MCF-7 and HCC1937, respectively, increased with decreasing hydrogel stiffness. Fig. 5(a) to Fig. 5(c) showed that the number of attached cells on HA-Tyr hydrogels appeared to be closely related to CD44 expression level (Fig. 4). Breast cancer cell adhesion on HA-Tyr hydrogel increased with increasing expression levels of CD44. These results strongly suggest that the cell adhesion on HA-Tyr hydrogels was dependent on the interactions between HA and CD44.

In contrast, only few BT-474 cells were attached onto the HA-Tyr gels (Fig. 5(d)). These results indicate that CD44 expression levels and/or stiffness of the hydrogel are factors that affect the adhesion of cancer cells onto the hydrogel. In general, the higher the expression level of CD44 and/or the lesser the hydrogel stiffness, the greater is the extent of cell adhesion to the hydrogel.

### Example 3

### Cancer Cell Proliferation

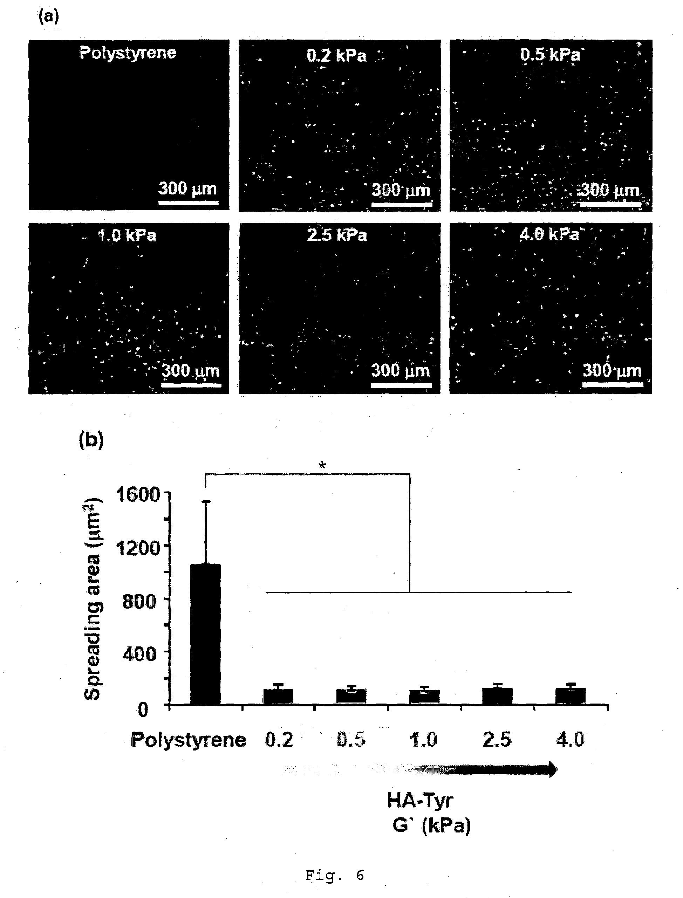

In order to determine the cell morphology of the attached MDA-MB-231 cells, an Olympus microscope camera equipped to IX71 inverted microscope (Tokyo, Japan) was used to obtain phase contrast microscopic images. Referring to Fig. 6(a), it was observed that all of the attached MDA-MB-231 cells were round regardless of gel stiffness, while many of the other cells were observed to be spread on the polystyrene culture flask.

Cell spreading area was analyzed using Image-Pro Plus image analysis software (from Maryland of the United States of America). The cell spreading area was shown in Fig. 6(b), which clearly revealed that the cell spreading area on HA-Tyr gels was significantly lower than that on polystyrene, while there was no significant difference in the cell spreading on the various HA-Tyr gels of various stiffness. These results indicated that the cell adhesion via interaction between CD44 and HA-Tyr gel could maintain the round-shaped MDA-MB-231 cells.

To evaluate the cell proliferation on the various gels (0.1, 0.2, 0.5, 1.0, and 4.0 kPa), 250 *µ*l of MDA-MB-231 cells in culture medium at cell density of 1.0 × 10⁵ cells/ml was seeded onto the gels. A cell culture well plate without the gel served as a comparison. The cells were incubated at 37°C in a humidified atmosphere of 5% CO₂. The culture medium was changed every 3 days. To evaluate the cell proliferation on hydrogels, the fluorescence measurement was performed using the CyQUANT® cell proliferation assay kit as described above.

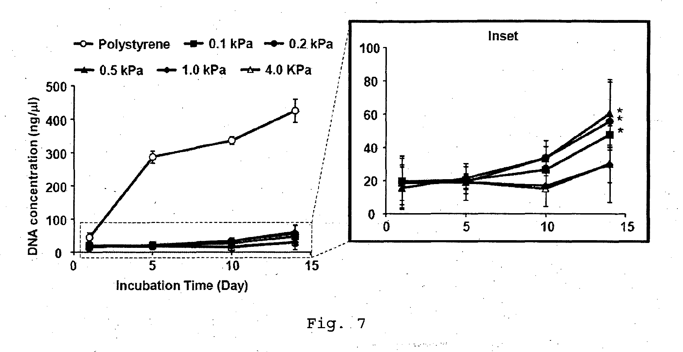

As shown in Fig. 7, the growth rate of MDA-MB-231 cells cultured on HA-Tyr gels was much slower than that cultured on culture plate. Only soft HA-Tyr gels with stiffness of 0.1 kPa, 0.2 kPa and 0.5kPa showed cell growth during 13 days, while no cell growth was observed from the HA-Tyr gels of other stiffness.

These results, including cell attachment and proliferation of HA-Tyr gels, were obviously different from those of culture plate. This suggests that CD44 expressed in MDA-MB-231 cells interacted with HA chains in the HA-Tyr gels.

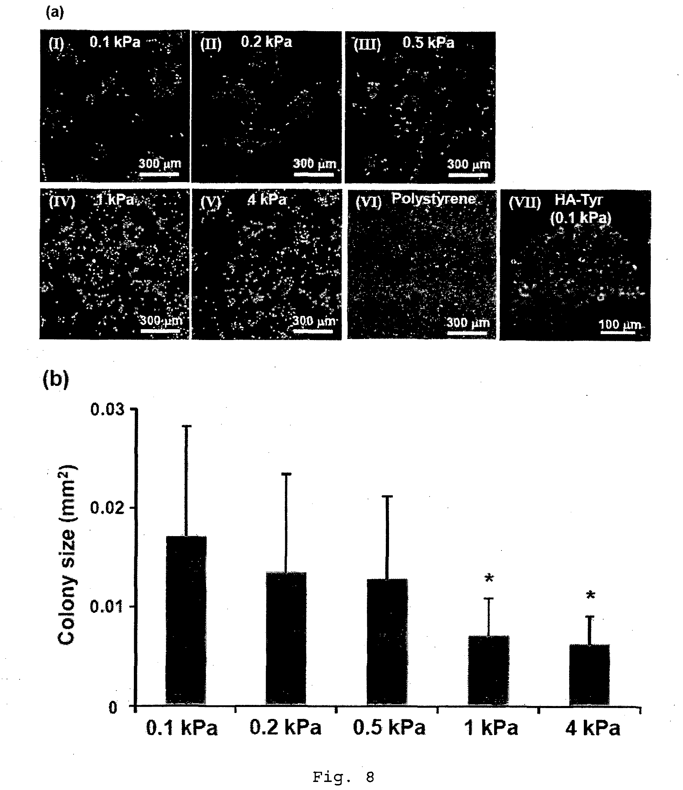

In order to investigate further the behavior of MDA-MB-231 breast cancer cells on HA-Tyr hydrogels, the colony formation on HA-Tyr hydrogels were evaluated (Fig. 8). Round-shaped MDA-MB-231 cells formed colonies on HA-Tyr hydrogels after 14 days (Fig. 8a I-V, XII), while no colonies were observed on polystyrene. The size of colony increased with decreasing the HA-Tyr hydrogel stiffness.

### Example 4

### Enhancement of expression levels of cancer stem cell markers by HA-Tyr hydrogel

The mRNA expression levels of Nanog, Sox-2 and EpCAM in MDA-MB-231 cells after 13 days of cultivation on HA-Tyr gel (0.2, 0.5, 1.0 and 4.0 kPa stiffness) were investigated. Additionally, the transcription levels of CD44s (CD44 standard), CD44v3-10 (CD44 variant isoform), CD44v8-10 (CD44 variant isoform), Sox-2, EpCAM and ALDHD1A1 genes in MDA-MB-231 cells on HA-Tyr hydrogels (0.1, 0.2, 0.5, 1.0 and 4.0k Pa stiffness) were also investigated.

Here, 250 *µ*l of MDA-MB-231 cells in culture medium at cell density of 1.0 × 10⁵ cells/ml was seeded onto the gels. A cell culture well plate without the gel served as a comparison. The cells were incubated at 37°C in a humidified atmosphere of 5% CO₂. The culture medium was changed every 3 days.

The MDA-MB-231 cells on the various HA-Tyr hydrogels were collected by treating with hyaluronidase (1,000 units/ml) and trypsin-EDTA for subsequent RNA extraction. The total RNA of MDA-MB-231 cell was extracted using a TRIzol (Life technologies, Singapore) after 13 days of culture. In order to prepare the cDNAs, total RNAs were incubated with RT reaction mixture (Thermo Scientific, China), which contains RT buffer, random hexamer primer, deoxynucleotide triphosphate (dNTP) mixture, RNase inhibitor and reverse transcriptase, followed by DNase treatment.

Real-time quantitative PCR was conducted using iQ5 multicolor RT PCR detection system (Bio-Rad laboratories, Singapore). The reaction mixtures (Life technologies, Singapore) contained 10 µL TaqMan PCR master mix, 1.0 µL of each primer, 2.0 µL of cDNA, and 7.0 µL of distilled water. The various primers used were Hs01075861_m1 (CD44 total), Hs01081473_m1 (CD44s), Hs01081480_m1 (CD44v3-v10), Hs01081475_m1 (CD44v8-v10)), Nanog (Hs02387400_s1), Sox-2 (Hs01053049_s1), EpCAM (Hs00901885_m1) and ALDH1A1 (Hs00946916_m1). The thermal profile used for PCR was 95°C for 20 seconds, forty cycles of 95°C for 3 seconds and 60°C for 20 seconds. The average threshold cycle (Ct) values of triplicate measurements were used in all subsequent calculations using the delta-delta Ct method, and results are presented as the fold change in gene expression normalized to an endogenous reference gene (GAPDH) and relative to the untreated control (polystyrene plates).

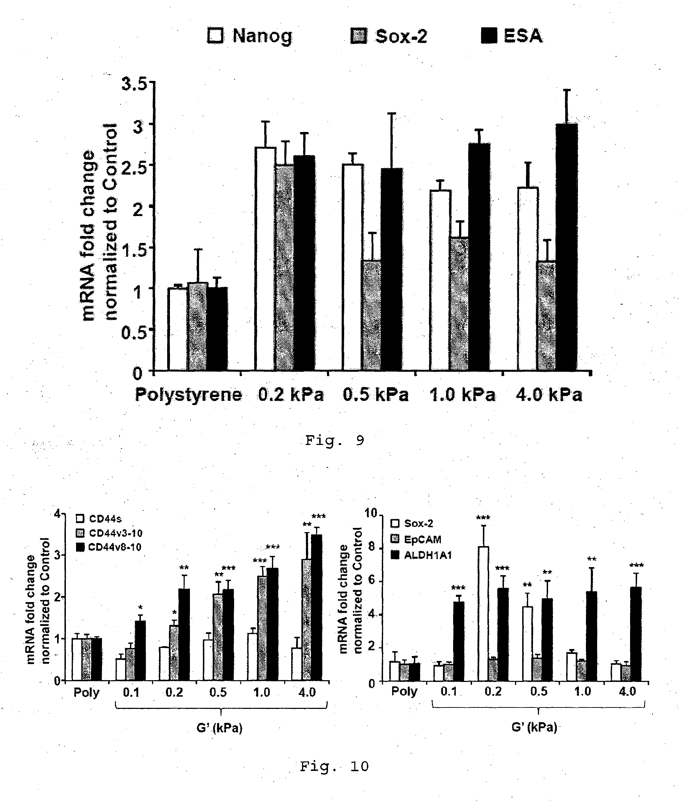

As shown in Fig. 9, it was observed that the mRNA expression levels of Nanog, Sox-2 and EpCAM from the HA-Tyr gel with stiffness of 0.2 kPa were significantly higher than those from the other culture plates. The other gels showed significantly higher mRNA expression level, except Sox-2. Nanog and Sox-2 are known to be sternness marker, and those transcription factors induce anti-apoptotic protein expression and chemoresistance. Further, the enhancement of ESA expression, which is one of the CSC markers, indicated that the MDA-MB-231 cells that adhered on the HA-Tyr gel are CSCs.

As shown in Fig. 10, the mRNA expression levels of CD44v3-10 and CD44v8-10 on HA-Tyr hydrogels were significantly higher than that on polystyrene, while the expression levels of CD44s were almost the same as that on polystyrene. Interestingly, the enhanced expression levels of CD44v3-10 and CD44v8-10 increased with increasing the HA-Tyr hydrogel stiffness (Fig. 10a). These results show that HA-Tyr hydrogel can enhance the expression levels of CD44v3-10 and CD44v8-10 in a hydrogel stiffness dependent manner, but not CD44s expression level. Therefore, the results strongly suggest that the HA-Tyr hydrogel induces malignant properties of breast cancer cells..

Furthermore, mRNA expression levels of Sox-2 and ALDH1A1 on HA-Tyr hydrogels were also significantly enhanced on HA-Tyr hydrogels compared to those on polystyrene (Fig. 10b). The expression level of ALDH1A1 was independent on hydrogel stiffness of HA-Tyr hydrogels. This result suggested that the CD44-HA interaction enhance ALDH1A1 expression in a stiffness independent manner. In the case of Sox-2, mRNA expression level was significantly increased on 0.2 and 0.5 kPa HA-Tyr hydrogel. Further, the enhancement of ALDH1A1 expression, which is one of the CSC markers, indicated that the MDA-MB-231 cells that adhered on the HA-Tyr hydrogel are induced CSC property.

Hence, it is shown that HA-based cell culture substrates can be useful for selection, culture and maintenance of CSCs.

### Example 5

Inhibition of MDA-MB-231 cell adhesion on HA-Tyr hydrogel using anti-CD44 antibody was evaluated. The MDA-MB-231 cells were washed with PBS and harvested from the cell culture flask using trypsin-EDTA. The detached cells were washed with RPMI-1640 medium without FBS, and were incubated with 30 *µ*g/ml anti-CD44 antibody on ice for 1 hour. A cell suspension without the anti-CD44 antibody served as a comparison. After washing with RPMI-1640 medium without FBS, 250 *µ*l of MDA-MB-231 cells in culture medium at cell density of 1.0 × 10⁵ cells/ml was seeded onto the 0.1 kPa HA-Tyr hydrogels, which were prepared in the 24-well plate in a similar manner as described above. The cells were incubated for 1 hour at 37°C in a humidified atmosphere of 5% CO₂. Folowing which, the media with unattached cells were aspirated and the wells were washed three times with PBS. To evaluate the number of attached cells on the HA-Tyr hydrogel, the fluorescence measurement was performed using the CyQUANT® cell proliferation assay kit as described above.

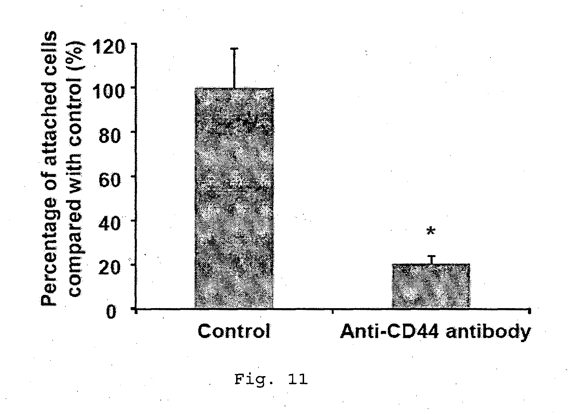

As shown in Fig. 11, pretreatment with anti-CD44 antibody drastically decreased the percentage of attached cells to 0.1 kPa HA-Tyr hydrogel compared with non-treated control. Hence, it can be seen that cell adhesion on HA-Tyr hydrogels was mostly mediated by the interaction of the HA chains with CD44 receptor.

### Example 6

### Cancer Cell Selection and Maintenance

To evaluate the cancer cell selection, transcription level of CD44s, CD44v3-10, CD44v8-10, EpCAM and ALHD1A1 genes in the attached MDA-MB-231 cells on HA-Tyr (0.1, 0.2, 0.5, 1.0 and 4.0 kPa) were analyzed. Two hundred fifty µl of breast cancer cells in culture medium at cell density of 1.0 × 10⁵ cells/ml was seeded onto the hydrogels. They were incubated for 1 h at 37°C in a humidified atmosphere of 5% CO₂, followed by washing three times with 500 µl of PBS to remove unattached cells. Hence, as compared to Example 4, an additional washing step was used here. The cells on the HA-Tyr hydrogels and polystyrene were harvested by incubating with hyaluronidase (1,000 units/ml) and trypsin-EDTA, respectively. The cell pellets were washed with PBS twice and were used for measurement of mRNA expression level.

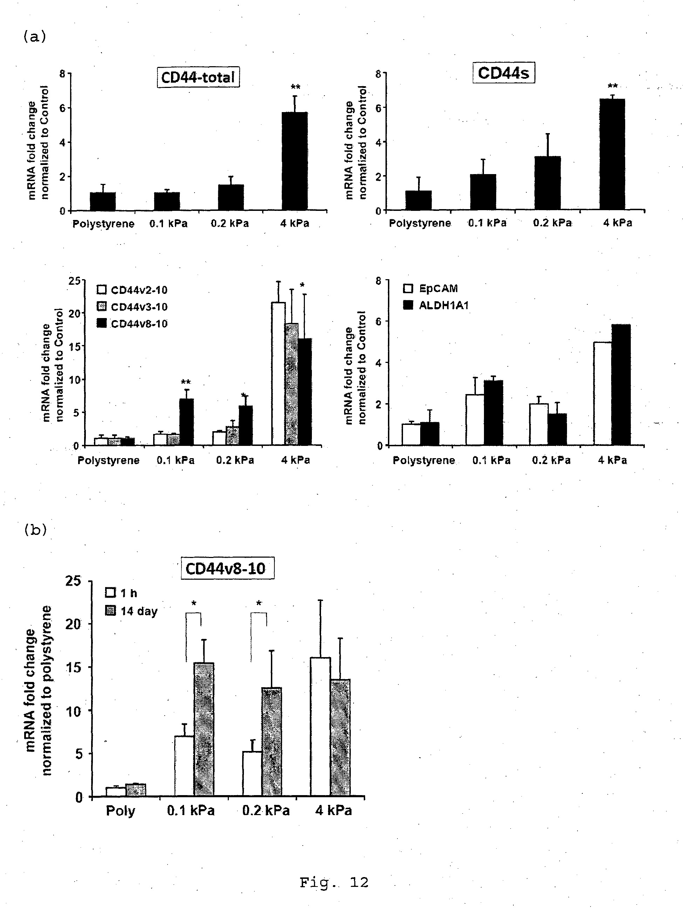

The mRNA expression levels of CSC markers such as CD44s, CD44v3-10, CD44v8-10, EpCAM and ALHD1A1 on 4 kPa HA-Tyr hydrogels were significantly higher than that on polystyrene (Fig 12(a)). Especially, CD44v8-10 level was significantly higher than that on polystyrene, even on soft (0.1 and 0.2 kPa) HA-Tyr hydrogels. CD44v8-10 have been well investigated the correlation with breast cancer metastasis due to enhancement of the resistance against reactive oxygen species (ROS). EpCAM and ALDH1A1 also are well known to correlate with CSC properties. Therefore, these results support the hypothesis that HA-Tyr hydrogels with tunable stiffness can be useful for selection of CSC cells. Furthermore, mRNA expression level of CD44v8-10 on 4 kPa HA-Tyr hydrogel was maintained after 14 days (Fig 12 (b)). In the case of 0.1 and 0.2 kPa HA-Tyr hydrogels, mRNA expression levels of CD44v8-10 were significantly enhanced after 14 days. These results indicate that the property of selected cancer cells was not only maintained but also enhanced on HA-Tyr hydrogels. In addition, the use of an additional washing step resulted in cells that highly expressed CD44, EpCAM and other markers.

### Example 7

### Chemoresistance of Cancer Cells

To evaluate chemoresistance, the cell viability of MDA-MB-231 on HA-Tyr gels was measured in the presence or absence of conventional anti-cancer drugs, such as cisplatin and doxorubicin. Here, 250 *µ*l of MDA-MB-231 cells in culture medium at cell density of 5.2 × 10³ cells/ml was seeded onto the gels (0.2, 0.5, 1.0, 2.5 and 4.0 kPa). The culture medium was changed every 3 days. After 10 days of cell seeding, 10-100 *µ*M cisplatin or doxorubicin was added to cultured cells on gels and incubated for 48 hours. Then, the cells were washed with PBS and the fluorescence intensity was measured using the AlamarBlue cell viability assay as described above. Cell viability in the presence of anti-cancer drugs was normalized to the untreated control groups (0 *µ*M).

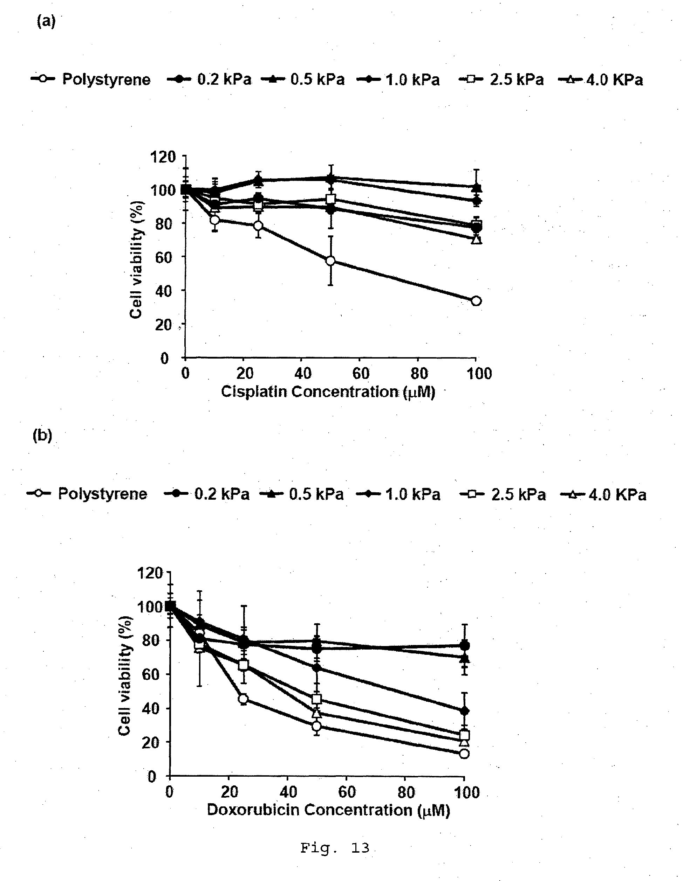

In the presence of cisplatin, MDA-MB-231 cell viability on the culture plate decreased in a dose-dependent manner (see Fig 13(a)). In contrast, HA-Tyr gels showed much higher cell viability in a test concentration range compared to cell culture plate. Notably, the cell viability was almost 100% at even 100 mM of cisplatin when HA-Tyr with 0.5 and 1 kPa.

A similar tendency was observed in the presence of doxorubicin (Fig. 13(b)). Approximately 80% viability was maintained on 0.2 and 0.5 kPa gels even in the presence of 100 *µ*M doxorubicin, while around 10 and 20% cell viability was observed on cell culture plate and HA-Tyr gels (2.5 and 4.0 kPa), respectively. These results clearly showed that the HA-Tyr, especially soft gel, significantly induced chemoresistance.

### Applications

The disclosed method can be used in cell culture applications to culture a desired cancer stem cell or cancer cell line that contains the cancer stem cell.

The disclosed method may be used to selectively separate and thereby isolate a desired cancer stem cell (or cancer cell line) from a plurality or mixture of various cancer cells.

The disclosed gel may be used to support the selection and growth of a desired cancer stem cell. The disclosed gel together with the cancer stem cells may be used as a drug screening platform for cancer therapy to determine anti-cancer drugs that the cancer stem cells are chemo-resistant to or which have a therapeutic effect on the cancer stem cells.

The disclosed method and gel may be used to selectively isolate cancer stem cells that contain a desired marker by receptor-ligand binding.

## Claims

1. A method for selectively separating a population of cancer stem cells from a plurality of cancer cells, comprising the steps of:
a. subjecting said plurality of cancer cells to a cell culture substrate, wherein said cell culture substrate is in the form of a gel comprising a conjugate of a glycosaminoglycan and a substituted phenalkylamine; and
b. allowing said cancer stem cells to interact with said cell culture substrate to thereby separate said cancer stem cells from said plurality of cancer cells,
wherein said cancer stem cells contain a marker that interacts with said glycosaminoglycan, the method further comprising removing cancer cells that are not attached to said cell culture substrate.

2. The method of claim, comprising selecting the storage modulus of said gel from a value in the range of 30 to 100,000 Pa.

3. The method of any one of claims 1 to 2, comprising selecting the stiffness of said gel from a value in the range of 0.1 to 100 kPa.

4. A method of screening drugs for a population of cancer stem cells comprising the step of culturing said cancer stem cells on or in a cell culture substrate, wherein said cell culture substrate is in the form of a gel comprising a conjugate of a glycosaminoglycan and a substituted phenalkylamine, and wherein said gel has a stiffness that is equal to or less than 100 kPa and wherein said cancer stem cells contain a marker that interacts with said glycosaminoglycan.

5. The method of any one of the preceding claims, wherein said glycosaminoglycan is a non-sulfated glycosaminoglycan.

6. The method of any one of the preceding claims, wherein said glycosaminoglycan is hyaluronic acid.

7. The method of any one of the preceding claims, wherein said substituted phenalkylamine is selected from the group consisting of phenmethylamine, phenethylamine, phenpropylamine, phenbutylamine and phenpentylamine.

8. The method of any one of the preceding claims, wherein said substituted phenalkylamineas is a tyramine.

9. The method of claim 8, wherein said tyramine is a meta-tyramine or a para-tyramine.

10. The method of any one of the preceding claims, comprising selecting the degree of substitution of said conjugate from a value in the range of 1 to 20.

11. The method of any one of the preceding claims, wherein said marker is a receptor for said glycosaminoglycan.

12. The method of any one of the preceding claims, wherein said marker is selected from the group consisting of CD44, Receptor for HA-mediated motility (RHAMM) and intracellular adhesion molecule-1 (ICAM-1) .

13. The method of any one of claims 4 to 12 wherein said cancer stem cells become resistant to an anti-cancer drug when the stiffness of said cell culture substrate is less than or equal to 100 kPa.

14. The method of claim 13, wherein said cancer stem cells have at least 70% viability in the presence of said anti-cancer drug.

## Patentansprüche

1. Verfahren zum selektiven Separieren einer Population von Krebsstammzellen aus seiner Vielzahl von Krebszellen, welches die folgenden Schritte umfasst:
a. Aussetzen der Vielzahl von Krebszellen einem Zellkultursubstrat, wobei das Zellkultursubstrat in der Form eines Gels vorliegt, welches ein Konjugat eines Glycosaminoglycan und eines substituierten Phenalkylamin umfasst; und
b. Erlauben den Krebsstammzellen mit dem Zellkultursubstrat zu wechselwirken, um hierdurch die Krebsstammzellen von der Vielzahl von Krebszellen zu separieren,
wobei die Krebsstammzellen einen Marker enthalten, der mit dem Glycosaminoglycan wechselwirkt, wobei das Verfahren weiterhin umfasst:
Entfernen von Krebszellen, die nicht an dem Zellkultursubstrat anhaften.

2. Verfahren nach Anspruch 1, welches weiterhin ein Auswählen des Speichermoduls des Gels aus Werten im Bereich von 30 bis 100.000 Pa umfasst.

3. Verfahren nach einem der Ansprüche 1 oder 2, umfassend:
Auswählen der Steifigkeit des Gels aus Werten im Bereich von 0,1 bis 100 kPa.

4. Verfahren zum Screenen von Medikamenten nach einer Population von Krebsstammzellen, welches den Schritt zum Kultivieren der Krebsstammzellen auf oder in einem Zellkultursubstrat umfasst, wobei das Zellkultursubstrat in der Form eines Gels vorliegt, das ein Konjugat eines Glycosaminoglycans und eines substituierten Phenalkylamin umfasst, und wobei das Gel eine Steifigkeit hat, die gleich oder kleiner 100 kPa ist und wobei die Krebsstammzellen einen Marker enthalten, der mit dem Glycosaminoglycan wechselwirkt.

5. Verfahren nach einem der vorhergehenden Ansprüche, wobei das Glycosaminoglycan ein nichtschwefeliges Glycosaminoglycan ist.

6. Verfahren nach einem der vorhergehenden Ansprüche, wobei das Glycosaminoglycan Hyaluronsäure ist.

7. Verfahren nach einem der vorhergehenden Ansprüche, wobei das substituierte Phenalkylamin aus der Gruppe ausgewählt ist, die aus Phenmethylamin, Phenethylamin, Phenpropylamin, Phenbutylamin, Phenpentylamin besteht.

8. Verfahren nach einem der vorhergehenden Ansprüche, wobei das substituierte Phenalkylamin ein Tyramin ist.

9. Verfahren nach Anspruch 8, wobei das Tyramin ein meta-Tyramin oder ein para-Tyramin ist.

10. Verfahren nach einem der vorhergehenden Ansprüche, welches ein Auswählen des Grades der Substitution des Konjugats aus Werten im Bereich 1 bis 20 umfasst.

11. Verfahren nach einem der vorhergehenden Ansprüche,
wobei der Marker ein Rezeptor für das Glycosaminoglycan ist.

12. Verfahren nach einem der vorhergehenden Ansprüche,
wobei der Marker aus der Gruppe ausgewählt ist, die aus CD44, einem Rezeptor für HA-vermittelte Motilität ("Receptor for HA-mediated motility" (RHAMM)) und einem intrazellulären Adhesionsmolekül-1 ("intracellular adhesion molecule-1" (ICAM-1)) ausgewählt ist.

13. Verfahren nach einem der Ansprüche 4 bis 12, wobei die Krebsstammzellen resistent gegenüber einem Krebsmedikament werden, wenn die Steifigkeit des Zellkultursubstrats kleiner oder gleich 100 kPa ist.

14. Verfahren nach Anspruch 13, wobei die Krebsstammzellen eine Überlebensfähigkeit von wenigstens 70 Prozent in Anwesenheit des Krebsmedikaments haben.

## Revendications

1. Procédé pour séparer sélectivement une population de cellules souches cancéreuses d'avec une pluralité de cellules cancéreuses, comprenant les étapes consistant à :
a. soumettre ladite pluralité de cellules cancéreuses à un substrat de culture cellulaire, lequel substrat de culture cellulaire est sous la forme d'un gel comprenant un conjugué d'un glycosaminoglycane et d'une phénalkylamine substituée ; et
b. laisser lesdites cellules souches cancéreuses interagir avec ledit substrat de culture cellulaire de façon à séparer ainsi lesdites cellules souches cancéreuses d'avec ladite pluralité de cellules cancéreuses,
dans lequel lesdites cellules souches cancéreuses contiennent un marqueur qui interagit avec ledit glycosaminoglycane, le procédé comprenant en outre l'élimination des cellules cancéreuses qui ne sont pas rattachées audit substrat de culture cellulaire.

2. Procédé selon la revendication 1, comprenant la sélection du module de stockage dudit gel dans la plage de valeurs allant de 30 à 100 000 Pa.

3. Procédé selon l'une quelconque des revendications 1 et 2, comprenant la sélection de la rigidité du gel dans la plage de valeurs allant de 0,1 à 100 kPa.

4. Procédé pour dépister des médicaments pour une population de cellules souches cancéreuses, comprenant l'étape consistant à cultiver lesdites cellules souches cancéreuses sur ou dans un substrat de culture cellulaire, dans lequel ledit substrat de culture cellulaire est sous la forme d'un gel comprenant un conjugué d'un glycosaminoglycane et d'une phénalkylamine substituée, et dans lequel ledit gel a une rigidité qui est égale ou inférieure à 100 kPa et dans lequel lesdites cellules souches cancéreuses contiennent un marqueur qui interagit avec ledit glycosaminoglycane.

5. Procédé selon l'une quelconque des revendications précédentes, dans lequel ledit glycosaminoglycane est un glycosaminoglycane non sulfaté.

6. Procédé selon l'une quelconque des revendications précédentes, dans lequel ledit glycosaminoglycane est l'acide hyaluronique.

7. Procédé selon l'une quelconque des revendications précédentes, dans lequel ladite phénalkylamine substituée est choisie dans le groupe constitué par la phénméthylamine, la phénéthylamine, la phénpropylamine, la phénbutylamine et la phénpentylamine.

8. Procédé selon l'une quelconque des revendications précédentes, dans lequel ladite phénalkylamine substituée est une tyramine.

9. Procédé selon la revendication 8, dans lequel ladite tyramine est une méta-tyramine ou une para-tyramine.

10. Procédé selon l'une quelconque des revendications précédentes, comprenant la sélection du degré de substitution dudit conjugué dans la plage de valeurs ayant de 1 à 20.

11. Procédé selon l'une quelconque des revendications précédentes, dans lequel ledit marqueur est un récepteur pour ledit glycosaminoglycane.

12. Procédé selon l'une quelconque des revendications précédentes, dans lequel ledit marqueur est choisi dans le groupe constitué par CD44, le récepteur de motilité à médiation par HA (RHAMM) et la molécule 1 d'adhésion intracellulaire (ICAM-1).

13. Procédé selon l'une quelconque des revendications 4 à 12, dans lequel lesdites cellules souches cancéreuses deviennent résistantes à un médicament anticancéreux quand la rigidité dudit substrat de culture cellulaire est inférieure ou égale à 100 kPa.

14. Procédé selon la revendication 13, dans lequel lesdites cellules souches cancéreuses ont une viabilité d'au moins 70 % en présence dudit médicament anticancéreux.
